# EUROPEAN PATENT SPECIFICATION

(11) **EP 3 911 300 B1**
(45) Date of publication and mention of the grant of the patent: **17.04.2024**
(21) Application number: 20701860.7
(22) Date of filing: 20.01.2020
(51) Int. Cl.: A61K 9/08, A61K 47/18, A61K 47/34, A61K 47/42

(54) **DRUG DELIVERY SYSTEM**
WIRKSTOFFFREISETZUNGSSYSTEM
SYSTÈME D'ADMINISTRATION DE MÉDICAMENT

(30) Priority: 18.01.2019 GB 201900728
(43) Date of publication of application: 24.11.2021
(73) Proprietor: The University of Birmingham, Birmingham B15 2TT (GB)
(72) Inventor: DE COGAN, Felicity, Edgbaston, Birmingham B15 2TT (GB); PEACOCK, Anna, Edgbaston, Birmingham B15 2TT (GB); NIKOI, Naa Dei, Edgbaston, Birmingham B15 2TT (GB)
(74) Representative: Marks & Clerk LLP
(86) International application number: PCT/GB2020/050108
(87) International publication number: WO 2020/148556

(56) References cited:
- EP-A2- 0 975 370
- WO-A1-2011/132826
- WO-A1-2018/090149
- WO-A2-2014/140890
- CA-A1- 3 015 467
- US-A1- 2005 232 981
- US-A1- 2011 053 829
- US-A1- 2016 287 663

## Description

The present disclosure relates to a drug delivery system, more particularly to a delivery system for transporting pharmaceutically active agents across, for example, the skin, the surface of the eye or mucosal membranes. Methods of using the delivery system are also provided.

### Background

There is a wide range of applications for delivering pharmaceutically active moieties across mucosal and epithelial surfaces. These include the treatment of acute conditions, such as burns, trauma and infection, and chronic treatments for conditions that include debilitating eye diseases such as glaucoma, age-related macular degeneration and retinal scarring after ocular surgery.

Uemura et al. (Circulation J. (2202), 66, 1155-1160) describe the translocation of short polymers of arginine into cultured vascular smooth muscle cells. Heptamers of arginine were especially rapidly transported into cells where they were used to study the effects of the polymers on nitric oxide synthesis. The polymers were observed to rapidly translocate through cytoplasmic and nuclear membranes efficiently.

Other so-called cell penetrating peptides have also been used to transport, for example, DNA or siRNA into cultured cells, but not in a more complex system of several layers of cells as found in transmembrane systems.

WO2015/114324 describes a system for transporting a pharmaceutically effective moiety across the dermis, cornea or skin of patients by using a polypeptide of up to 20 amino acids in length.

The present disclosure has been devised with these issues in mind.

### Summary

According to a first aspect of the present disclosure there is provided a drug delivery system comprising a pharmaceutically effective moiety and a polyamine or amine moiety, for use in the treatment of eye disease, wherein the polyamine or amine moiety does not comprise two or more contiguous basic amino acid residues and wherein the polyamine or amine moiety has a molecular weight of no more than 500 g/mol and wherein the pharmaceutically effective moiety and the polyamine or amine moiety are constituted by separate molecules or are non-covalently bonded..

It will be understood that the pharmaceutically effective moiety may be a peptide comprising amino groups (e.g. a polyamine). It will also be understood that in such embodiments the drug delivery system of the present disclosure must include another polyamine or amine moiety as a carrier agent, wherein said polyamine or amine moiety does not comprise two or more contiguous basic amino acid residues.

As disclosed herein, the pharmaceutically effective moiety may also be referred to as a pharmaceutically active moiety.

As used herein, a polyamine moiety is a molecule, or a part of a molecule, which comprises at least two amino groups. The polyamine moiety may have at least three or at least four amino groups. In some embodiments, the polyamine moiety has no more than ten, no more than eight, no more than six or no more than five amino groups.

The polyamine moiety may comprise primary (-NH₂), secondary (-NRH) or tertiary (-NR₂) amino groups, or a mixture thereof.

In some embodiments, the polyamine moiety comprises at least one secondary amino group. The polyamine moiety may comprise two, three, four, five or more secondary amino groups.

The polyamine moiety may be straight- or branched-chain, or a cyclic molecule.

The polyamine moiety may terminate in a primary amino group at one or more of its ends. In some embodiments the polyamine moiety is a straight- or branched-chain molecule having a primary amino group at each end.

In some embodiments, the polyamine moiety comprises no more than one basic amino acid residue.

In some embodiments, the polyamine moiety does not comprise two or more contiguous amino acid residues.

In some embodiments, the polyamine moiety does not comprise any basic amino acid residues.

In some embodiments, the polyamine moiety does not comprise any amino acid residues.

In some embodiments, the polyamine moiety does not comprise any amido groups.

As used herein, the term "basic amino acid residue" refers to histidine, arginine or lysine.

In some embodiments, each amino group of the polyamine moiety is separated from the other amino group(s) by a spacer moiety.

The (or each) spacer moiety may independently be a substituted or unsubstituted, straight- or branched-chain, alkyl, alkenyl or alkynyl group. The (or each) spacer moiety may independently comprise from 1 to 10, from 2 to 8 or from 3 to 6 carbon atoms. In some embodiments one or more of the spacer moieties comprises more than 10 carbon atoms. In some embodiments one or more of the spacer moieties comprises no more than 20, no more than 15, no more than 12 or no more than 10 carbon atoms.

The polyamine moiety may comprise two or more spacer moieties. Two or more (or even all) of the spacer moieties may be the same, or they may be different. It will thus be understood that any individual spacer may be the same as or different to any other spacer in the polyamine moiety.

In some embodiments, at least one spacer moiety is an alkyl group having from two to eight or from three to six carbon atoms. In some embodiments, each spacer moiety is an alkyl group having from two to eight or from three to six carbon atoms. The alkyl group may be straight-chain. The alkyl group may be unsubstituted.

In some embodiments, the polyamine moiety is selected from the group consisting of spermine, spermidine, putrescine, cadaverine, diethylenetriamine (DETA), triethylenetetraamine (TETA), tetraethylenepentamine (TEPA), pentaethylenehexamine (PEHA), pentamethyldiethylenetriamine (PMDTA), 1,4,7-triazacyclononane, cyclen, cyclam, tris(2-aminoethyl)amine, 1,1,1-tris(aminomethyl)ethane, N,N'-bis(3-aminopropyl)-1,3-propanediamine, bis(3-aminopropyl)amine, N-methylethylenediamine, 1,2-bis(3-aminopropylamino)ethane, guanidine, L-carnosine and 3-(methylamino)propylamine.

In other embodiments, the drug delivery system comprises the pharmaceutically effective moiety covalently linked to one of the compounds listed in the immediately preceding paragraph.

In some embodiments, the amine moiety which acts as the carrier agent is an amine moiety comprising a single amino group, instead of a polyamine moiety. The single amino moiety may be straight- or branched-chain, or a cyclic molecule.

The single amino moiety may terminate in a primary amino group at one of its ends. In some embodiments, the single amino moiety is a straight- or branched-chain molecule having a primary amino group at one end.

In some embodiments, the single amino moiety comprises a secondary or tertiary amino group.

In some embodiments, the single amino moiety comprises a secondary amino group and two aliphatic groups. The two aliphatic groups may be identical, or the single amino moiety may comprise two different aliphatic groups. In some embodiments, one or each of the aliphatic groups is an alkyl chain comprising from 1 to 10, from 2 to 8 or from 3 to 6 carbon atoms. In some embodiments, one or each of the aliphatic groups is an alkyl chain comprising 2, 3, 4, 5 or 6 carbon atoms.

In some embodiments, the single amino moiety comprises a tertiary amino group and three aliphatic groups. In some embodiments, the three aliphatic groups are identical to each other. Alternatively, two of the aliphatic groups may be identical to each other. Alternatively, all three aliphatic groups may be different to each other. In some embodiments, one or each of the aliphatic groups is an alkyl chain comprising from 1 to 10, from 2 to 8 or from 3 to 6 carbon atoms. In some embodiments, one or each of the aliphatic groups is an alkyl chain comprising 2, 3, 4, 5 or 6 carbon atoms. In some embodiments, one or each of the aliphatic groups is an alkenyl group comprising from 1 to 10, from 2 to 8 or from 3 to 6 carbon atoms and at least one carbon-carbon double bond. In some embodiments, one or each of the aliphatic groups is an alkenyl group comprising 2, 3, 4, 5 or 6 carbon atoms and at least one carbon-carbon double bond.

In some embodiments, the single amino moiety is selected from the group consisting of dibutylamine, triethylamine and triethyleneamine.

In some embodiments, the drug delivery system comprises two or more different polyamine or amine moieties. In some embodiments, the drug delivery system comprises two or more different polyamine moieties. In some embodiments, the drug delivery system comprises two or more different single amino moieties. In some embodiments, the drug delivery system comprises a combination of at least one polyamine moiety and at least one single amino moiety.

In some embodiments the polyamine or amine moiety has a molecular weight of no more than 500, no more than 400, no more than 300, no more than 200 or no more than 150 g/mol.

The pharmaceutically active moiety and the polyamine or amine moiety may be constituted by separate molecules, or they may form different parts of the same molecule.

Thus, in some embodiments, the pharmaceutically effective moiety is bound to the polyamine or amine moiety by a covalent or non-covalent (e.g. ionic) bond.

The pharmaceutically effective moiety may be directly bound to the polyamine or amine moiety by a covalent bond. Alternatively, the pharmaceutically effective moiety may be covalently bound to the polyamine or amine moiety via a linker. Such linkers are generally known in the art for the attachment of compounds to proteins or other pharmaceutically active moieties.

Suitable linkers may be based on any of the following functionalities: succinimidyl succinate, N-hydroxy succinimide, succinimidyl propionate, succinimidyl butanoate, propionaldehyde, acetaldehyde, tresylate, triazine, vinyl sulfone, benzotriazole carbonate, maleimide, pyridyl sulfide, iodoacetamide, succimidyl carbonate or avidin/biotin.

In some embodiments, the pharmaceutically effective moiety is not bound to the polyamine or amine moiety, but is simply mixed with the polyamine or amine moiety in the drug delivery system.

The pharmaceutically active moiety may be, for example, an antibody (e.g. ranibizumab, bevacizumab or aflibercept), a peptide (such as a polypeptide), a protein, a glycoprotein, a nucleic acid (such as an siRNA), or a small molecule (e.g. glucose).

It will be understood that the term "antibody", as used herein, includes any form of antibodies, including domain antibodies, Fc, Fab and F(ab')2 fragments, single chain variable region fragments (scFV) or IgA/B/C/D/E/F and especially IgG (any of subtypes 1,2 3 or 4). Where reference is made herein to an antibody, it will be appreciated that this includes biopharmaceuticals. These biopharmaceuticals may include humanised antibodies, domains and fragments of antibodies, chimeric antibodies, bi-specifc antibodies, antibody-drug conjugates, non-immunoglobulin protein scaffolds including, but not restricted to adnexins, darpins, camelids, shark variable domains and non-protein domains including but not restricted to aptamers.

The antibody may be human or humanised versions of any of the antibodies described herein. If that reference antibody is not human or humanised, then a humanised or human version thereof is preferred. The generation of humanised or human antibody from, for example, murine antibody is generally well known in the art.

In some embodiments, the pharmaceutically effective moiety is a steroid, an anti-oxidant (such as lutein or zeazanthin), an anti-dyslipidemic agent, a PPAR (peroxisome proliferator-activated receptor)-agonist, an anti-amyloid agent, an inhibitor of lipofuscin, a visual light cycle modulator, a dark adaptation agent, a neuroprotector, an apoptosis inhibitor, a necrosis inhibitor, a C-reactive protein inhibitor, a complement system inhibitor, an immunosuppressant, a matrix metaloproteinase modulator, an anti-angiogenic agent, an anti-inflammatory compound or an antimicrobial compound.

In some embodiments, the pharmaceutically effective moiety is protein or a peptide. In some embodiments, the pharmaceutically effective moiety is lutein or bevacizumab.

The pharmaceutically active moiety may have a molecular weight of no more than 500 kDa, 400 kDa, 300 kDa, 200 kDa or 150 kDa.

In some embodiments, the ratio of the pharmaceutically effective moiety to the polyamine or amine moiety in the drug delivery system is from 1:100 to 100:1, from 1:50 to 50:1, from 1:30 to 30:1, from 1:10 to 10:1, from 1:5 to 5:1, from 1:2 to 2:1, from 1:1.5 to 1.5:1, or 1:1, by weight.

The pharmaceutically active moiety and the polyamine or amine moiety may be present in the drug delivery system in solution, suspension or dispersion. For example, the drug delivery system may be in the form of a liquid composition, with the pharmaceutically active moiety and the polyamine or amine moiety dissolved, suspended or dispersed in a pharmaceutically acceptable liquid or buffer. The drug delivery system may be formulated as a liquid, a spray, a cream, a lotion or a gel.

The drug delivery system of the present disclosure may be suitable for transporting the pharmaceutically active moiety across single or multi-layered membranes, for example, the cornea of the surface of the eye, mucous membranes or skin. Thus, the drug delivery system may be suitable for transmucosal and/or transepithelial drug delivery.

For example, the system may be provided as in the form of eye drops, for example in a saline solution for applying to the surface of the eye to allow the pharmaceutically active moiety to be transported across the membrane of the eye into the eye itself.

Similarly, it may be provided in the form of drops, for example for applying to the mucous membrane of the nose, or indeed as an ointment for applying to the surface of the nose. The system may be provided in the form of a suppository to allow the pharmaceutically active moiety to be absorbed through the mucous membranes of the rectum, vagina, or urethra. Typically such suppositories comprise a greasy base, such as cocoa butter, in which the active ingredient and other excipients are dissolved. The grease will melt at body temperature to release the active ingredients. Other suppositories may be made from a water soluble base such as polyethylene glycol, glycerin or gelatin.

The drug delivery system may also be provided in the form of a patch which can be applied to the surface of the skin. Transdermal patches are medicated adhesive patches that are placed on the skin to deliver a specific dose of medication through the skin and into the blood stream. The pharmaceutically active moiety and the polyamine or amine moiety may be provided within, for example, an adhesive layer. The adhesive layer not only serves to adhere various layers together, along with the entire system to the skin, but is also responsible for the releasing of the pharmaceutically active moiety and the polyamine or amine moiety onto the skin. Multi-layer drug systems are also known which use a separate layer for control of release of drug from a reservoir onto the skin.

The drug delivery system of the present disclosure may be used in combination with one or more additional penetration enhancers.

The penetration enhancer may be selected from but not exclusively, for example, polyethylene glycol, fatty acid esters, diacids and monomethyl esters.

According to a further example of the present disclosure, there is provided the drug delivery system of the first aspect of the present disclosure or embodiments thereof, for use as a medicament.

According to a yet further example of the present disclosure, there is provided the drug delivery system of the first aspect of the present disclosure or embodiments thereof, for use in the treatment of a disease or condition selected from eye disease, burns, infection, and trauma.

Trauma may be traumatic injury to the skin or eye. Traumatic injury to the eye may, for example, be caused by abrasion of the cornea, or penetration of the eye by a foreign object.

Eye diseases which may be treated by the present disclosure include glaucoma, proliferative vitreoretinopathy, age related macular degeneration, retinal scarring (e.g. after ocular surgery) and retinitis pigmentosa.

The infection may be a bacterial, viral, or fungal infection.

In a further example, the present disclosure provides a method of treating a disease or condition selected from eye disease, burns, trauma, and infection, the method comprising administering a pharmaceutically effective amount of the drug delivery system of the present disclosure to a patient in need thereof.

The drug delivery system may be administered by application to the skin, the surface of the eye or a mucous membrane, as appropriate. The skilled person would be capable of determining an effective quantity of the drug delivery system to be administered, depending on factors including the disease or condition being treated, the concentration of the pharmaceutically active moiety in the drug delivery system, and the patient.

The present disclosure also provides a method of transporting a pharmaceutically effective moiety across the skin, the surface of an eye or a mucous membrane, the method comprising applying to the skin, the eye or the membrane a drug delivery system according to the present disclosure.

Embodiments of the present disclosure will now be described by way of example, and with reference to the accompanying figures in which:
Figure 1 is a bar chart showing the fluorescent units measured following delivery of a fluorescent peptide across a membrane in the presence and absence of a polyamine carrier agent;
Figure 2 is bar chart showing the amount of lutein which is carried across a membrane in the presence and absence of a polyamine carrier agent;
Figure 3 is a bar chart showing the delivery of a fluorescent peptide into a retinal tissue in ex *vivo* porcine eyes;
Figure 4 is a bar chart showing the delivery of a fluorescent peptide into aqueous humour in ex *vivo* porcine eyes;
Figure 5 is a bar chart showing the delivery of bevacizumab into the posterior segment of ex *vivo* porcine eyes using spermine;
Figure 6 is a bar chart showing the delivery of bevacizumab into the posterior segment of ex *vivo* porcine eyes using spermidine;
Figure 7 is a bar chart showing the amount of fluorescent peptide delivered across a membrane in the presence of various polyamine or amine moieties;
Figure 8 is a bar chart showing the amount of lutein delivered across a membrane in the presence of various polyamine or amine moieties;
Figure 9 is a bar chart showing the amount of bevacizumab delivered across a membrane in the presence of various polyamine or amine moieties.

### Example 1: Delivery of peptide across a biological membrane using spermine

This experiment tested the ability of a polyamine carrier agent to transport a peptide across a biological membrane, as a model of a drug delivery system according to the present disclosure.

### Methods

A water-soluble, 29-amino acid peptide developed at the University of Birmingham was used. The peptide was tagged with 5(6)-carboxyfluorescein (FAM), which is a fluorescent dye having an absorption wavelength of 495nm and an emission wavelength of 517 nm.

The polyamine carrier agent used was spermine. Spermine is a short linear polyamine that is found in human bodily fluids. It has four amino groups, including two primary amines, one at each terminus of the molecule, and two secondary amines. The secondary amino groups are separated by an unsubstituted alkyl spacer moiety having four carbon atoms. Each terminal primary amine group is spaced from its nearest secondary amine group by an unsubstituted alkyl spacer moiety having three carbon atoms.

The tagged peptide was formulated at 2.5 mg/mL in PBS (phosphate-buffered saline). Spermine was formulated at 60 mg/400 µL in PBS. The peptide and spermine solutions were then mixed at a ratio of 1:1. To provide controls, the peptide solution only and the spermine solution only were separately mixed with PBS at a 1:1 ratio.

The ability of the tagged peptide to cross a biological membrane was then tested. The membrane used was the epithelial membrane from a chicken egg, which has a thickness of up to 2 mm. Fine tweezers were used to remove the membrane from the egg shell. A circular section of membrane was cut, having a diameter of 1.5-2 cm. The membrane was washed in deionized water. The circular section of membrane was fluted (in the same way that a piece of filter paper would be fluted).

50 µL of deionized water was placed into the well of a 96-well plate, and the fluted membrane was placed in the well. 50 µL of the peptide-spermine mixture (or 50 µL of the control mixture) was applied to the membrane, and a timer was set for 60 seconds. After 60 seconds, the membrane was removed from the well. The fluorescence of the water in the well plate was read using a Fluostar^{®} optima plate reader.

### Results

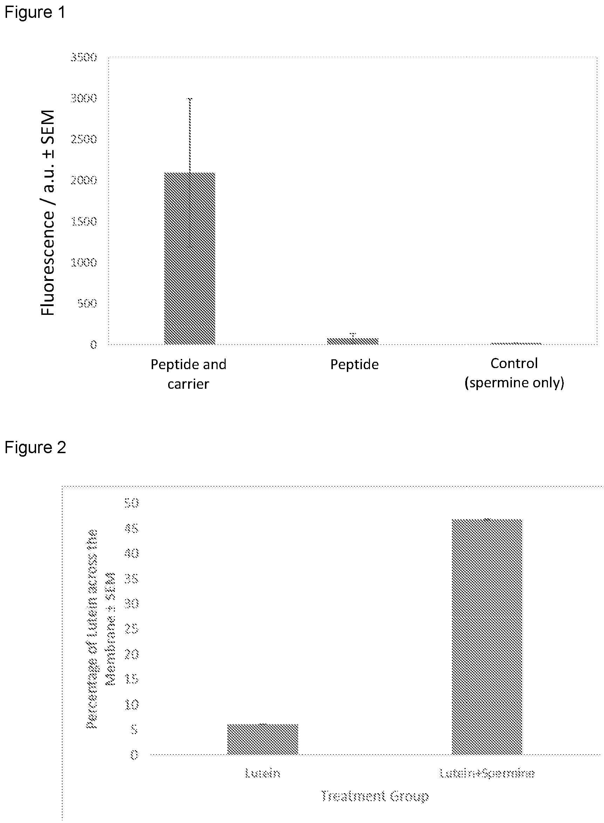

The results of the experiment are shown in Figure 1. The y axis of the bar chart shows fluorescent units. In the presence of the spermine, 2093 fluorescent units were measured, whereas in the absence of the spermine only 77 units were measured. These results show that the presence of the polyamine carrier significantly enhances the transport of the fluorescent peptide across the membrane in. Based on the fluorescence measurement of the initial solution it is estimated that approximately 5% of the tagged protein was transported across the membrane during the 60 second test period.

### Example 2: Delivery of lutein across a biological membrane using spermine

A further test was carried out using lutein using the same methodology as described in Example 1. Lutein is a naturally occurring carotenoid that reduces the progression of age-related macular degeneration and cataracts. Lutein has a characteristic absorbance which can be measured using a plate reader, and thus it was not necessary to label the lutein with FAM. The absorbance of the solution which had crossed the membrane was measured and compared to the absorbance of the stock solution.

The results are shown in Figure 2. It can be seen that in the absence of the carrier agent, only about 6% of the lutein crossed the membrane, whereas in the presence of the carrier agent about 46% of the lutein was transported across the membrane.

### Example 3: Delivery of peptide into eye tissue using spermine

### Methods

5(6)-carboxyflureoscein tagged peptide was used as in previous experiments. The peptide was formulated at 2.5 mg/mL in PBS. Spermine was formulated at 60 mg/400 µL in PBS. The peptide and spermine solutions were mixed at a 1:1 ratio. For the controls (peptide only or spermine only) the stock solutions were mixed with PBS at a 1:1 ratio. The solutions were made up fresh for the experiment.

Fresh adult porcine eyes were supplied by a butcher and placed in the cold room on arrival. The eyes were placed in 6-well plates (one plate for the peptide-spermine mixture, one plate for each control) and irrigated with PBS. Residual PBS was removed from the cornea with a pipette. Each group contained four eyes (four repeats).

A 50 µL drop of the peptide-spermine mixture, or control solution, was applied, circling the cornea (touching the sclera), finishing on the cornea. The eyes were incubated in a dark humidified chamber at room temperature for 20 minutes. After the incubation time was complete the eyes were washed with deionised water to remove any remaining drop from the front of the eye and prevent contamination when dissecting.

The aqueous humour was first removed using a needle, which was inserted directly through the cornea. The aqueous humour collected was injected into a labelled 96-well plate. 50 µL from the injected solution was then pipetted into a clean well in the same 96-well plate so that on reading the plate the same volume/path length was used for each group/repeat.

The vitreous was removed using tweezers and placed into a 15 mL tube before using a sonicating wand for 15 seconds to blend the sample. 1 mL of the sample was then pipetted into a clean well in a 12-well plate so that on reading the plate the same volume/path length was used for each group/repeat.

The retina was then removed and placed into a 15 mL tube before using a sonicating wand for 15 seconds to blend the sample. 100 µL of the sample was pipetted into a clean well in a 96-well plate so that on reading the plate the same volume/path length was used for each group/repeat.

The aqueous humour, retina and vitreous samples were then scanned on a Fluostar^{®} Omega spectrometer to detect fluorescence with excitation and emission filters 485/520 nm respectively and gain values of 500 and 1800.

Serial dilutions of samples in a 96-well and 12-well plate using the same sample volumes as those used above should provide a means to quantitatively determining the amount of peptide being delivered.

### Results

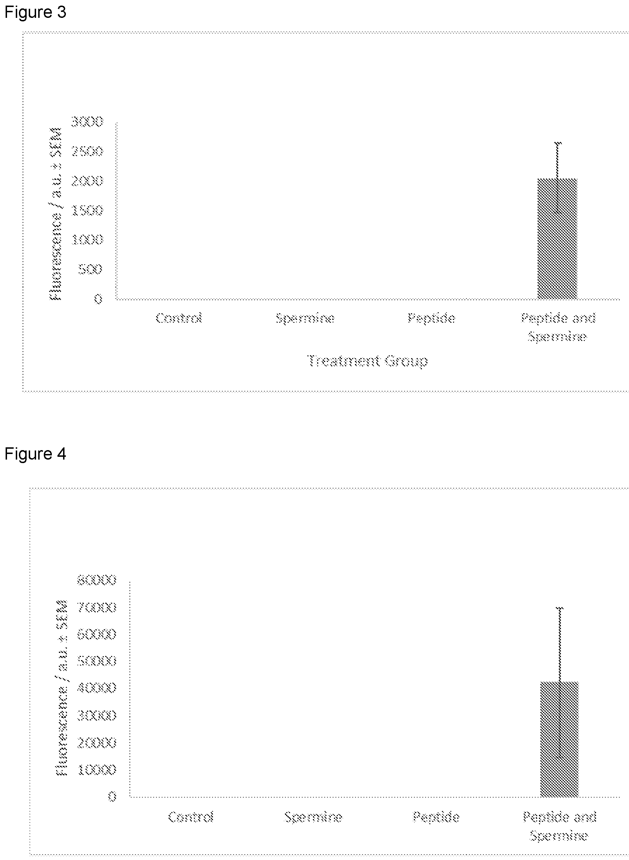

The results of the experiments are shown in Figures 3 and 4. In both the retina (Figure 3) and the aqueous humour (Figure 4) the levels of fluorescence was significantly higher than the control groups, indicating the presence of the peptide in the sample.

However, no peptide was detected in the vitreous. This is believed to be because the peptide crossed the sclera/conjunctiva directly into the retina, without passing through the vitreous.

These results show that the presence of the carrier protein enabled the protein to be transported across the cornea into the aqueous humour, and across the sclera and conjuctiva into the retina. This demonstrates the ability of a polyamine to function as a carrier agent in a transepithelial delivery system.

### Example 4: Delivery of bevacizumab into eye tissue using spermine

Further tests on porcine eyes was carried out with fluorescence-labelled bevacizumab. Bevacizumab (also known as Avastin^{®}) is a cancer treatment drug which can also be used to treat age-related macular degeneration. The same methodology as described in Example 3 was used, but with various incubation times.

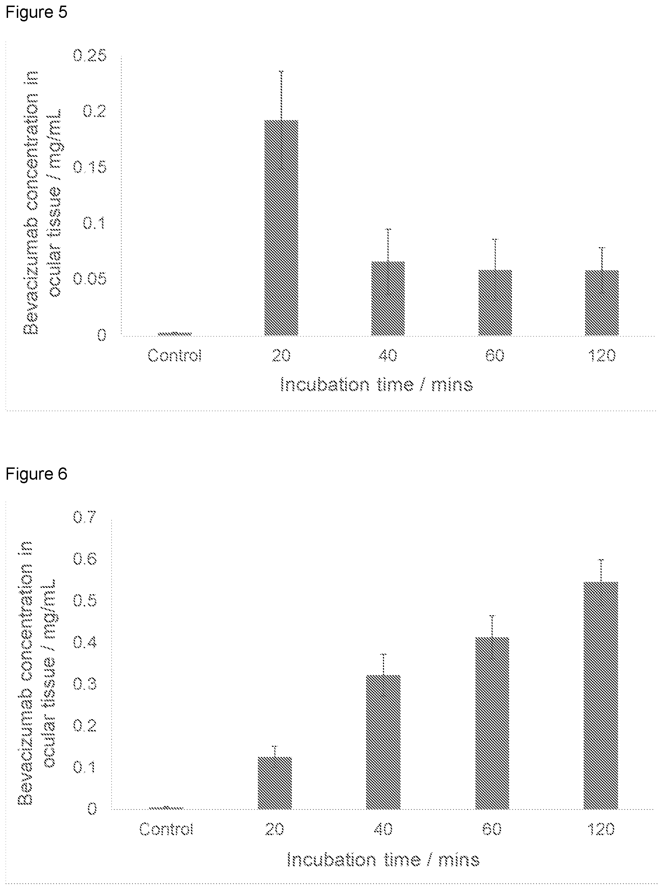

The amount of bevacizumab transported into the posterior segment of the eye is shown in Figures 5 and 6, where the results in Figure 5 were obtained using spermine as a polyamine carrier agent and the results in Figure 6 were obtained using spermidine as a polyamine carrier agent. It can be seen that, using spermine, the maximum amount of transport of bevacizumab into the eye tissue is achieved within 20 minutes, and allowing a longer incubation time did not result in higher delivery. By contrast, using spermidine, the amount of bevacizumab transported into the eye tissue increased with increasing incubation time.

### Example 5: Delivery of pharmaceutical agents across a biological membrane in the presence of various polyamine or amine moieties

### Peptide

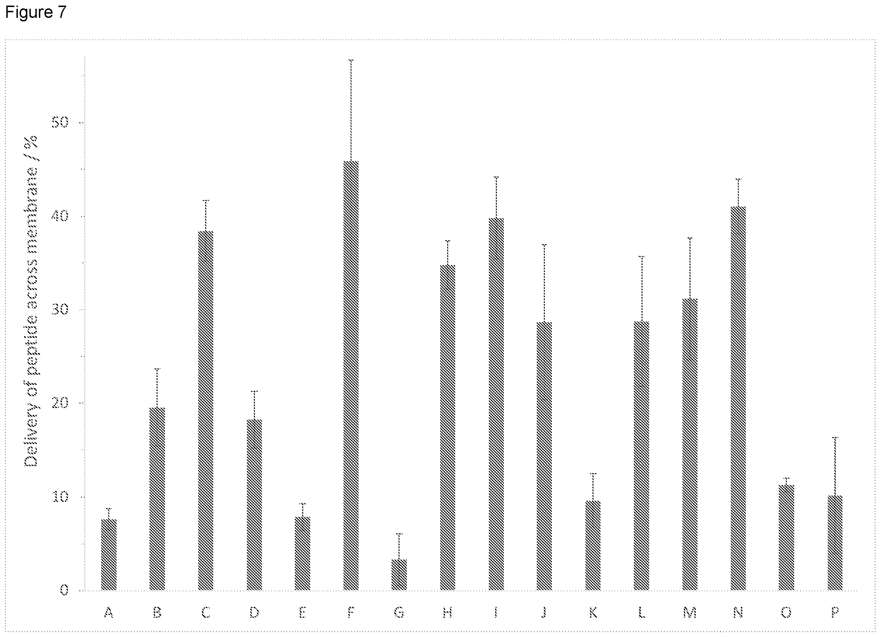

Further tests were carried out with the fluorescent peptide using the same methodology as described in Example 1, with a range of different polyamine or amine moieties. The results are shown in Figure 7 and listed in Table 1 below.

| **Table 1** | | |
|---|---|---|
| **Test** | **Polyamine moiety** | **% peptide transported across membrane** |
| A | None (control) | 7.6 |
| B | 1,2-Bis(3-aminopropylamino)ethane | 19.6 |
| C | Bis(3-aminopropyl)amine | 38.4 |
| D | Diethylenetriamine | 18.2 |
| E | Guanidine | 7.9 |
| F | N,N'-Bis(3-aminopropyl)-1,3-propanediamine | 45.9 |
| G | N,N-Diisopropylethylenediamine | 3.3 |
| H | N-Methylethylenediamine | 34.8 |
| I | Spermidine | 39.8 |
| J | Spermine | 28.7 |
| K | Cyclam | 9.6 |
| L | Tetraethylenepentamine | 28.8 |
| M | Triethyleneamine | 31.2 |
| N | Tris(2-aminoethyl)amine | 41.0 |
| O | Tyramine | 11.3 |
| P | Urea | 10.2 |

In the absence of any polyamine or amine moiety, only 7.6% of the peptide was transported across the membrane. Guanidine, N,N-diisopropylethylenediamine, cyclam, tyramine and urea were not found to provide a significant improvement in the amount of peptide transported across the membrane. On the other hand, using bis(3-aminopropyl)amine, N,N'-bis(3-aminopropyl)-1,3-propanediamine, spermidine and tris(2-aminoethyl)amine as polyamine carrier agents resulted in five times more peptide being transported across the membrane compared with the control test. 1,2-Bis(3-aminopropylamino)ethane, diethylenetriamine, N-methylethylenediamine, spermine, tetraethylenepentamine as polyamine carrier agents were also found to be effective for delivering peptide across the membrane, as well as triethyleneamine (a single amino moiety carrier agent), with twice as much peptide transported across the membrane as in the control sample.

### Lutein

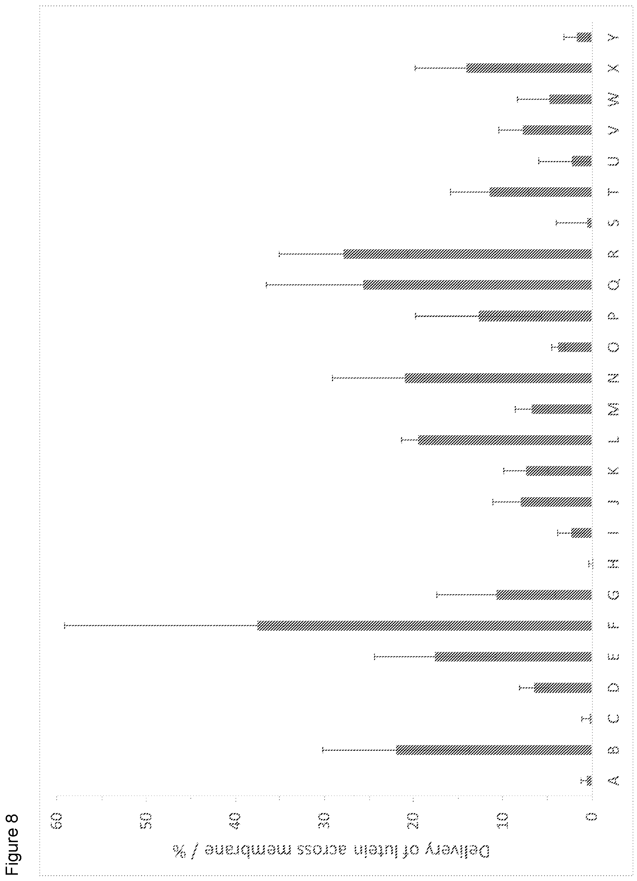

Further tests were carried out with lutein using the same methodology as described in Example 1, with a range of different polyamine or amine moieties. The results with various polyamine or amine moieties are shown in Figure 8 and listed in Table 2 below.

| **Table 2** | | |
|---|---|---|
| **Test** | **Polyamine moiety** | **% peptide transported across membrane** |
| A | None (control) | 0.5 |
| B | 1,2-Bis(3-aminopropylamino)ethane | 22.0 |
| C | 1,3-Diaminopropane | 0.2 |
| D | 3-(Methylamino)propylamine | 6.5 |
| E | Bis(3-aminopropyl)amine | 17.6 |
| F | Cadaverine | 37.5 |
| G | Dibutylamine | 10.7 |
| H | Diethylenetriamine | 0 |
| I | Ethylenediamine | 2.3 |
| J | Guanidine | 8.0 |
| K | L-Carnosine | 7.4 |
| L | N,N'-Bis(3-aminopropyl)-1,3-propanediamine | 19.5 |
| M | N-Methylethylenediamine | 20.9 |
| N | N,N-Diisopropylethylenediamine | 6.7 |
| O | Pentaethylenehexamine | 3.7 |
| P | Putrescine | 12.7 |
| Q | Spermidine | 25.7 |
| R | Spermine | 27.8 |
| S | Cyclam | 0.5 |
| T | Tetraethylenepentamine | 11.5 |
| U | Triethylamine | 2.2 |
| V | Triethyleneamine | 7.7 |
| W | Tris(2-aminoethyl)amine | 4.7 |
| X | Tyramine | 14.1 |
| Y | Urea | 1.7 |

In the absence of any polyamine or amine moiety, only 0.5% lutein was transported across the membrane. 1,3-Diaminopropane, diethylenetriamine, ethylenediamine, pentaethylenehexamine, cyclam, triethylamine and tris(2-aminoethyl)amine did not significantly improve the amount of lutein transported across the membrane. Using 1,2-bis(3-aminopropylamino)ethane, cadaverine, spermidine or spermine as polyamine carrier agents, or N-methylethylenediamine as a single amino carrier agent, resulted in a substantial increase in the amount of lutein transported across the membrane (>20%). 3-(Methylamino)propylamine, bis(3-aminopropyl)amine, dibutylamine, guanidine, L-carnosine, N,N'-bis(3-aminopropyl)-1,3-propanediamine, N,N-diisopropylethylamine putrescine, tetraethylenepentamine, triethyleneamine and tyramine also showed good effectiveness (>5%).

### Bevacizumab (Avastin^{®})

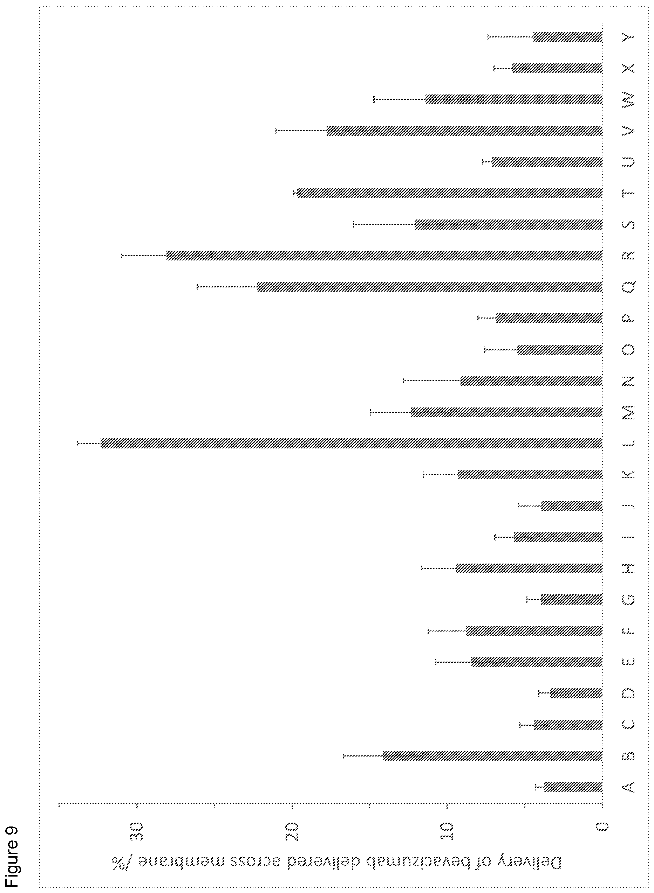

Further tests were carried out with fluorescence-labelled bevacizumab using the same methodology as described in Example 1, with a variety of different polyamine or amine moieties. The results are shown in Figure 9 and listed in Table 3 below.

| **Table 3** | | |
|---|---|---|
| **Test** | **Polyamine moiety** | **% peptide transported across membrane** |
| A | None (control) | 3.8 |
| B | 1,2-Bis(3-aminopropylamino)ethane | 14.1 |
| C | 1,3-Diaminopropane | 4.4 |
| D | 3-(Methylamino)propylamine | 3.4 |
| E | Bis(3-aminopropyl)amine | 8.5 |
| F | Cadaverine | 8.8 |
| G | Dibutylamine | 4.0 |
| H | Diethylenetriamine | 9.4 |
| I | Ethylenediamine | 5.7 |
| J | Guanidine | 4.0 |
| K | L-Carnosine | 9.3 |
| L | N,N'-Bis(3-aminopropyl)-1,3-propanediamine | 32.4 |
| M | N-Methylethylenediamine | 12.4 |
| N | N,N-Diisopropylethylenediamine | 9.1 |
| O | Pentaethylenehexamine | 5.5 |
| P | Putrescine | 6.9 |
| Q | Spermidine | 22.3 |
| R | Spermine | 28.1 |
| S | Cyclam | 12.1 |
| T | Tetraethylenepentamine | 19.7 |
| U | Triethylamine | 7.1 |
| V | Triethyleneamine | 17.8 |
| W | Tris(2-aminoethyl)amine | 11.4 |
| X | Tyramine | 5.8 |
| Y | Urea | 4.5 |

In the absence of any polyamine or amine moiety, only 4% bevacizumab was transported across the membrane. Using 1,3-diaminopropane, 3-(methylamino)propylamine, dibutylamine, ethylenediamine, guanidine, pentaethylenehexamine, putrescine, triethylamine, tyramine and urea did not significantly improve the amount of bevacizumab transported across the membrane. Using 1,2-bis(3-aminopropylamino)ethane, N,N'-bis(3-aminopropyl)-1,3-propanediamine, N-methylethylenediamine, spermidine, spermine, cyclam or tetraethylenepentamine as polyamine carrier agents, or triethyleneamine as a single amino carrier agent, resulted in more than three times the amount of bevacizumab being transported across the membrane, compared to the control sample. Bis(3-aminopropyl)amine, cadaverine, diethylenetriamine, L-carnosine, N,N-diisopropylethylenediamine, tris(2-aminoethyl)amine and tyramine also showed good effectiveness, with more than twice as much bevacizumab being transported across the membrane, compared to the control sample.

## Claims

1. A drug delivery system comprising a pharmaceutically effective moiety and a polyamine or amine moiety, for use in the treatment of eye disease, wherein the polyamine or amine moiety does not comprise two or more contiguous basic amino acid residues and wherein the polyamine or amine moiety has a molecular weight of no more than 500 g/mol and wherein the pharmaceutically effective moiety and the polyamine or amine moiety are constituted by separate molecules or are non-covalently bonded.

2. The drug delivery system for use of claim 1, wherein the polyamine or amine moiety is a polyamine moiety, which has at least three amino groups.

3. The drug delivery system for use of claim 2, wherein the polyamine moiety comprises at least one secondary amino group.

4. The drug delivery system for use of claim 2 or claim 3, wherein the polyamine moiety is a straight- or branched-chain molecule having a primary amino group at each end.

5. The drug delivery system for use of any one of claims 2 to 4, wherein each amino group is separated from each other amino group by a spacer moiety.

6. The drug delivery system for use of claim 5, wherein one or more of the spacer moieties is a substituted or unsubstituted, straight- or branched-chain, alkyl, alkenyl or alkynyl group.

7. The drug delivery system for use of claim 6, wherein one or more of the spacer moieties is a straight-chain, unsubstituted alkyl group having from 1 to 10 carbon atoms.

8. The drug delivery system for use of claim 1, wherein the polyamine or amine moiety is an amine moiety comprising a single secondary or tertiary amino group.

9. The drug delivery system for use of any preceding claim, wherein the polyamine or amine moiety does not comprise any amino acid residues.

10. The drug delivery system for use of any preceding claim, wherein the polyamine or amine moiety does not comprise any amido groups.

11. The drug delivery system for use of claim 1, wherein the polyamine or amine moiety is a polyamine moiety selected from the group consisting of spermine, spermidine, putrescine, cadaverine, diethylenetriamine (DETA), triethylenetetramine (TETA), tetraethylenepentamine (TEPA), pentaethylenehexamine (PEHA), pentamethyldiethylenetriamine (PMDTA), 1,4,7-triazacyclononane, cyclen, cyclam, tris(2-aminoethyl)amine, 1,1,1-tris(aminomethyl)ethane, N,N'-bis(3-aminopropyl)-1,3-propanediamine, bis(3-aminopropyl)amine, N-methylethylenediamine, 1,2-bis(3-aminopropylamino)ethane, guanidine, L-carnosine and 3-(methylamino)propylamine.

12. The drug delivery system for use of claim 1, wherein the polyamine or amine moiety is an amine moiety selected from the group consisting of dibutylamine, triethylamine and triethyleneamine.

13. The drug delivery system for use of any preceding claim, wherein the drug delivery system comprises two or more different polyamine or amine moieties.

14. The drug delivery system for use of any preceding claim, wherein the pharmaceutically effective moiety is a peptide, a protein, an antibody, a glycoprotein, a small molecule or a nucleic acid.

15. The drug delivery system for use of any preceding claim, wherein the pharmaceutically active moiety has a molecular weight of no more than 500 kDa.

## Patentansprüche

1. System zur Verabreichung von Arzneimitteln, umfassend einen pharmazeutisch wirksamen Anteil und einen Polyamin- oder Aminanteil zur Verwendung bei der Behandlung von Augenkrankheiten, wobei der Polyamin- oder Aminanteil nicht zwei oder mehr zusammenhängende basische Aminosäurereste umfasst und wobei der Polyamin- oder Aminanteil ein Molekulargewicht von nicht mehr als 500 g/Mol aufweist und wobei der pharmazeutisch wirksame Anteil und der Polyamin- oder Aminanteil durch getrennte Moleküle gebildet werden oder nicht kovalent gebunden sind.

2. System zur Verabreichung von Arzneimitteln zur Verwendung nach Anspruch 1, wobei der Polyamin- oder Aminanteil ein Polyaminanteil ist, der mindestens drei Aminogruppen besitzt.

3. System zur Verabreichung von Arzneimitteln zur Verwendung nach Anspruch 2, wobei der Polyaminanteil mindestens eine sekundäre Aminogruppe umfasst.

4. System zur Verabreichung von Arzneimitteln zur Verwendung nach Anspruch 2 oder 3, wobei der Polyaminanteil ein gerad- oder verzweigtkettiges Molekül ist, das an jedem Ende eine primäre Aminogruppe besitzt.

5. System zur Verabreichung von Arzneimitteln zur Verwendung nach einem der Ansprüche 2 bis 4, wobei jede Aminogruppe von jeder anderen Aminogruppe durch einen Abstandhalteranteil getrennt ist.

6. System zur Verabreichung von Arzneimitteln zur Verwendung nach Anspruch 5, wobei ein oder mehrere der Abstandhalteranteile eine substituierte oder unsubstituierte, gerad- oder verzweigtkettige Alkyl-, Alkenyl- oder Alkinylgruppe sind.

7. System zur Verabreichung von Arzneimitteln zur Verwendung nach Anspruch 6, wobei eine oder mehrere der Abstandhalteranteile eine geradkettige, unsubstituierte Alkylgruppe mit 1 bis 10 Kohlenstoffatomen sind.

8. System zur Verabreichung von Arzneimitteln zur Verwendung nach Anspruch 1, wobei der Polyamin- oder Aminanteil ein Aminanteil ist, der eine einzelne sekundäre oder tertiäre Aminogruppe umfasst.

9. System zur Verabreichung von Arzneimitteln zur Verwendung nach einem der vorhergehenden Ansprüche, wobei der Polyamin- oder Aminanteil keine Aminosäurereste umfasst.

10. System zur Verabreichung von Arzneimitteln zur Verwendung nach einem der vorhergehenden Ansprüche, wobei der Polyamin- oder Aminanteil keine Amidogruppen umfasst.

11. System zur Verabreichung von Arzneimitteln nach Anspruch 1, wobei der Polyamin- oder Aminanteil ein Polyaminanteil ist, der ausgewählt ist aus der Gruppe bestehend aus Spermin, Spermidin, Putrescin, Cadaverin, Diethylentriamin (DETA), Triethylentetramin (TETA), Tetraethylenpentamin (TEPA), Pentaethylenhexamin (PEHA), Pentamethyldiethylentriamin (PMDTA), 1,4,7-Triazacyclononan, Cyclen, Cyclam, Tris(2-aminoethyl)amin, 1,1,1-Tris(aminomethyl)ethan, N,N'-Bis(3-aminopropyl)-1,3-Propandiamin, Bis(3-aminopropyl)amin, N-Methylethylendiamin, 1,2-Bis(3-aminopropylamino)ethan, Guanidin, L-Carnosin und 3-(Methylamino)propylamin.

12. System zur Verabreichung von Arzneimitteln zur Verwendung nach Anspruch 1, wobei der Polyamin- oder Aminanteil ein Aminanteil ist, der aus der Gruppe ausgewählt ist, bestehend aus Dibutylamin, Triethylamin und Triethylenamin.

13. System zur Abgabe von Arzneimitteln zur Verwendung nach einem der vorhergehenden Ansprüche, wobei das System zur Abgabe von Arzneimitteln zwei oder mehrere unterschiedliche Polyamin- oder Aminanteile umfasst.

14. System zur Abgabe von Arzneimitteln zur Verwendung nach einem der vorhergehenden Ansprüche, wobei der pharmazeutisch wirksame Anteil ein Peptid, ein Protein, ein Antikörper, ein Glykoprotein, ein kleines Molekül oder eine Nukleinsäure ist.

15. System zur Abgabe von Arzneimitteln zur Verwendung nach einem der vorhergehenden Ansprüche, wobei der pharmazeutisch wirksame Anteil ein Molekulargewicht von nicht mehr als 500 kDa aufweist.

## Revendications

1. Système d'administration de médicament comprenant une portion pharmaceutiquement efficace et une portion polyamine ou amine, pour utilisation dans le traitement d'une maladie oculaire, la portion polyamine ou amine ne comprenant pas deux résidus d'acide aminé basiques contigus ou plus et dans lequel la portion polyamine ou amine présente un poids moléculaire d'au plus 500 g/mol et dans lequel la portion pharmaceutiquement active et la portion polyamine ou amine sont constitués de molécules séparées ou sont liées de manière non-covalente.

2. Système d'administration de médicament pour utilisation selon la revendication 1, dans lequel la portion polyamine ou amine est une portion polyamine, qui présente au moins trois groupes amino.

3. Système d'administration de médicament pour utilisation selon la revendication 2, dans lequel la portion polyamine comprend au moins un groupe amino secondaire.

4. Système d'administration de médicament pour utilisation selon la revendication 2 ou la revendication 3, dans lequel la portion polyamine est une molécule à chaîne droite ou ramifiée présentant un groupe amino primaire à chaque extrémité.

5. Système d'administration de médicament pour utilisation selon l'une quelconque des revendications 2 à 4, dans lequel chaque groupe amino est séparé de chaque autre groupe amino par une portion espaceur.

6. Système d'administration de médicament pour utilisation selon la revendication 5, dans lequel une ou plusieurs des portions espaceurs sont un groupe alkyle, alcényle ou alcynyle à chaîne droite ou ramifiée, substitué ou non substitué.

7. Système d'administration de médicament pour utilisation selon la revendication 6, dans lequel une ou plusieurs des portions espaceurs sont un groupe alkyle non substitué à chaîne droite présentant de 1 à 10 atomes de carbone.

8. Système d'administration de médicament pour utilisation selon la revendication 1, dans lequel la portion polyamine ou amine est une portion amine comprenant un groupe amino secondaire ou tertiaire unique.

9. Système d'administration de médicament pour utilisation selon l'une quelconque des revendications précédentes, dans lequel la portion polyamine ou amine ne comprend pas de résidu d'acide aminé.

10. Système d'administration de médicament pour utilisation selon l'une quelconque des revendications précédentes, dans lequel la portion polyamine ou amine ne comprend pas de groupes amido.

11. Système d'administration de médicament pour utilisation selon la revendication 1, dans lequel la portion polyamine ou amine est une portion polyamine sélectionnée dans le groupe constitué de spermine, spermidine, putrescine, cadavérine, diéthylènetriamine (DETA), triéthylènetétramine (TETA), tétraéthylènepentamine (TEPA), pentaéhylènehexamine (PEHA), pentaméthyldiéthylènetriamine (PMDTA), 1,4,7-triazacyclononane, cyclène, cyclame, tri(2-aminoéthyl)amine, 1,1,1-tris(aminométhyl)éthane, N,N'-bis(3-aminopropyl)-1,3-propanediamine, bis(3-aminopropyl)amine, N-méthyléthylènediamine, 1,2-bis(3-aminopropylamino)éthane, guanidine, L-carnosine et 3-(méthylamino)propylamine.

12. Système d'administration de médicament pour utilisation selon la revendication 1, dans lequel la portion polyamine ou amine est une portion amine sélectionnée dans le groupe constitué de dibutylamine, de triéthylamine et de triéthylèneamine.

13. Système d'administration de médicament pour utilisation selon l'une quelconque des revendications précédentes, dans lequel le système d'administration de médicament comprend deux portions polyamine ou amine différentes ou plus.

14. Système d'administration de médicament pour utilisation selon l'une quelconque des revendications précédentes, dans lequel la portion pharmaceutiquement efficace est un peptide, une protéine, un anticorps, une glycoprotéine, une petite molécule ou un acide nucléique.

15. Système d'administration de médicament pour utilisation selon l'une quelconque des revendications précédentes, dans lequel la portion pharmaceutiquement active présente un poids moléculaire d'au plus 500 kDa.
